# EUROPEAN PATENT SPECIFICATION

(11) **EP 2 894 219 B1**
(45) Date of publication and mention of the grant of the patent: **28.03.2018**
(21) Application number: 13834839.6
(22) Date of filing: 04.09.2013
(51) Int. Cl.: A61L 27/60, A61L 27/38, A61L 27/36, C12N 5/0784, C12N 1/00, C12N 5/07

(54) **ARTIFICIAL SKIN TISSUE, ARTIFICIAL SKIN MODEL AND MANUFACTURING METHOD THEREFOR**
KÜNSTLICHES HAUTGEWEBE, KÜNSTLICHES HAUTMODELL UND HERSTELLUNGSVERFAHREN DAFÜR
TISSU CUTANÉ ARTIFICIEL, MODÈLE DE PEAU ARTIFICIELLE ET LEUR PROCÉDÉ DE FABRICATION

(30) Priority: 04.09.2012 JP 2012194398
(43) Date of publication of application: 15.07.2015
(73) Proprietor: Osaka University, Suita-shi, Osaka 565-0871 (JP); BioMedical Technology HYBRID Co., Ltd., Kagoshima 890-0065 (JP)
(72) Inventor: AKASHI, Mitsuru, Suita-shi Osaka 565-0871 (JP); MATSUSAKI, Michiya, Suita-shi Osaka 565-0871 (JP); NAWANO, Masaaki, Kagoshima-shi Kagoshima 890-0065 (JP); HASHIMOTO, Koji, Iyo-gun Ehime 791-2101 (JP); SHIRAKATA, Yuji, Toon-shi Ehime 791-0295 (JP)
(74) Representative: Wilkins, Christopher George
(86) International application number: PCT/JP2013/073833
(87) International publication number: WO 2014/038599

(56) References cited:
- WO-A1-2012/133629
- DE-A1-102006 006 461
- JP-A- 2004 166 685
- JP-A- 2010 172 247
- JP-A- 2011 004 935
- JP-A- 2012 115 254
- JP-A- 2012 205 516
- JP-B2- 2 773 058
- US-A- 5 667 961
- AKIHIRO NISHIGUCHI ET AL: "Rapid Construction of Three-Dimensional Multilayered Tissues with Endothelial Tube Networks by the Cell-Accumulation Technique", ADVANCED MATERIALS, WILEY - V C H VERLAG GMBH & CO. KGAA, DE, vol. 23, no. 31, 16 August 2011 (2011-08-16), pages 3506-3510, XP002753112, ISSN: 0935-9648, DOI: 10.1002/ADMA.201101787 [retrieved on 2011-07-04]
- MICHIYA MATSUSAKI ET AL.: 'Kekkan Oyobi Limph-kan'yo Network o Yusuru Hifu Model no Kochiku' REGENERATIVE MEDICINE vol. 11, no. SUPL 2, 16 May 2012, page 164, XP008178572
- TADASHI UCHINO ET AL.: 'Construction of Three-dimensional Human Skin Model Involving Dendritic Cells and Its Application to Skin Sensitization Test' JOURNAL OF THE PHARMACEUTICAL SOCIETY OF JAPAN vol. 128, no. 1, 2008, pages 45 - 50, XP055235018
- LAMMERS,G. ET AL.: 'A molecularly defined array based on native fibrillar collagen for the assessment of skin tissue engineering biomaterials' BIOMATERIALS vol. 30, 2009, pages 6213 - 6220, XP026574959

## Description

### Technical Field

The present disclosure relates to an artificial skin tissue, an artificial skin model, and methods for manufacturing them.

### Background Art

In research and development of substances that are directly applied to humans, such as cosmetics, medicines, and quasi drugs, evaluation tests of the substances, including a drug effect test, a pharmacological test, and a safety test are important. Conventionally, these tests have been performed using animals such as mice and rats. In recent years, however, reconsideration of animal experiments is required in terms of animal welfare. Therefore, various methods have been proposed to produce a three-dimensional tissue of cells in vitro (see, e.g., Patent Document 1). For instance, one technique for producing artificial tissues uses a cell-accumulation method using single cells coated with fibronectin-gelatin nanofilms (Non-patent Document 1).

As one of alternative methods of animal experiments, e.g., there is an in vitro test using a cultured skin model. For this reason, many studies have been conducted on cultured skin or the like (see, e.g., Patent Documents 2 and 3). For instance, a whole skin equivalent comprising a collagen matrix and dendritic cells has been developed for use in testing the sensitizing effect of compounds (Patent Document 4).

### Prior Art Documents

### Patent Documents

Patent Document 1: JP 2012-115254 A
Patent Document 2: Japanese Patent No. 2773058
Patent Document 3: WO 2005/087286
Patent Document 4: DE 10 2006 006461

### Non-patent Documents

Non-patent Document 1: Nishiguchi et al., (2011) Advanced Materials, Vol. 23, No. 31, pages 3506-3510.

### Disclosure of Invention

### Problem to be Solved by the Invention

It is known that dendritic cells such as Langerhans cells located in an epidermal layer play a major role in skin immunity. Therefore, when the immune response of the skin is evaluated, it is important for an artificial skin model used for the evaluation to include dendritic cells such as Langerhans cells. However, e.g., the cultured skin of Patent Documents 2 and 3 does not include dendritic cells such as Langerhans cells. Accordingly, the immune response of the skin cannot be sufficiently evaluated with this cultured skin. Thus, there has been a growing demand for an artificial skin model including dendritic cells.

The present disclosure provides an artificial skin tissue and an artificial skin model that include dendritic cells, and novel methods capable of manufacturing the artificial skin tissue and the artificial skin model.

### Means for Solving Problem

An artificial skin tissue is disclosed herein, wherein the artificial skin tissue includes the following: a dermal tissue layer that includes an extracellular matrix component and layered cells; a basal layer that includes type IV collagen and is formed on the dermal tissue layer; and an epidermal layer that is formed on the basal layer. At least one of the dermal tissue layer and the epidermal layer includes dendritic cells.

In one or more embodiments, the present disclosure relates to a method for manufacturing an artificial skin tissue, which includes the following: preparing coated cells in which a cell surface is coated with a coating film containing an extracellular matrix component; forming a dermal tissue layer by culturing the coated cells, so that the coated cells are layered three-dimensionally; forming a basal layer including type IV collagen on the dermal tissue layer by bringing type IV collagen into contact with the dermal tissue layer; and forming an epidermal layer by arranging epidermal cells on the basal layer, wherein at least one of the dermal tissue layer and the epidermal layer includes dendritic cells.

A method for manufacturing an artificial skin model is disclosed herein, which includes the following: forming a dermal tissue layer by culturing coated cells in which a cell surface is coated with a coating film containing an extracellular matrix component, so that the coated cells are layered; forming a basal layer including type IV collagen on the dermal tissue layer by bringing type IV collagen into contact with the dermal tissue layer; and forming an epidermal layer by arranging epidermal cells on the basal layer. At least one of the dermal tissue layer and the epidermal layer includes dendritic cells.

In one or more embodiments, the present disclosure relates to an artificial skin tissue manufactured by the method of the invention including the following: a dermal tissue layer that includes an extracellular matrix component and layered cells; a basal layer that includes type IV collagen and is formed on the dermal tissue layer; and an epidermal layer that is formed on the basal layer, wherein the dermal tissue layer and optionally the epidermal layer includes dendritic cells, wherein cells in the dermal tissue layer are layered via the extracellular matrix component, and wherein the dermal tissue layer includes lymphatic vessels.

### Effects of the Invention

The present disclosure provides an artificial skin tissue including dendritic cells and a novel method capable of manufacturing the artificial skin tissue as defined above. A method capable of manufacturing an artificial skin model including dendritic cells is also disclosed herein.

### Brief Description of Drawings

FIGS. 1A and 1B show an example of confocal laser scanning microscope (CLSM) images of an artificial skin model in Example 1.
FIGS. 2A to 2D show an example of confocal laser scanning microscope (CLSM) images of an artificial skin model in Example 2.
FIG. 3 is an example of a graph showing the relationship between the amount of LPS added and the amount of IL-6 produced from a dermal tissue layer.
FIG. 4 is an example of a graph showing the relationship between the amount of LPS added and the amount of IL-6 produced from a dermal tissue layer.
FIG. 5 shows an example of a confocal laser scanning microscope (CLSM) image taken 30 hours after the addition of LPS.

### Description of the Invention

The present disclosure is based on the findings that coated cells in which a cell surface is coated with a coating film containing an extracellular matrix component are cultured to form a dermal tissue layer, and a basal layer including type IV collagen (also referred to as "collagen IV" in the following) is formed between the dermal tissue layer and an epidermal layer, thereby preparing an artificial skin model in which the dermal tissue layer has a laminated structure with excellent long-term stability, and dendritic cells are introduced.

The "artificial skin tissue" in the present specification refers to an artificial skin tissue that reproduces or imitates a human skin structure, particularly a structure including an epidermal layer and a dermal tissue layer, and an environment of the structure. In one or more embodiments, the artificial skin tissue of the present disclosure can be manufactured by the method of the invention and includes a dermal tissue layer that includes an extracellular matrix component and layered cells, a basal layer that includes collagen IV and is formed on the dermal tissue layer, and an epidermal layer that is formed on the basal layer, wherein the dermal tissue layer and optionally the epidermal layer includes dendritic cells, wherein cells in the dermal tissue layer are layered via the extracellular matrix component, and wherein the dermal tissue layer includes lymphatic vessels. In one or more embodiments, the artificial skin tissue of the present disclosure can be preferably a skin grafting material, or an experimental tool or a test tool that can be used for drug evaluation such as a drug effect test, a pharmacological test, or a safety test of a test substance on the skin.

The "artificial skin model" disclosed in the present specification refers to an artificial skin model that reproduces or imitates a human skin structure, particularly a structure including an epidermal layer and a dermal tissue layer, and an environment of the structure. The artificial skin model disclosed herein includes a dermal tissue layer that includes an extracellular matrix component and layered cells, a basal layer that includes collagen IV and is formed on the dermal tissue layer, and an epidermal layer that is formed on the basal layer. At least one of the dermal tissue layer and the epidermal layer includes dendritic cells. The artificial skin model can be preferably an experimental tool or a test tool that can be used for drug evaluation such as a drug effect test, a pharmacological test, or a safety test of a test substance on the skin. The artificial skin model of the present disclosure is more preferably an artificial skin model manufactured by the manufacturing method of the present disclosure. In the following description of the present specification, unless otherwise noted, the "artificial skin tissue" and the "artificial skin model" are collectively referred to as the "artificial skin model".

In one or more embodiments, the "dendritic cells" in the present specification may include dermal dendritic cells, Langerhans cells, etc. The dendritic cells may also include precursor cells of these cells

In one or more embodiments, the artificial skin model of the present disclosure may include, other than the dendritic cells and lymphatic vessels, a further tissue ancillary organ included in the skin and/or cells constituting the tissue ancillary organ. Examples of the further tissue ancillary organ include a blood vessel, a sebaceous gland, a sweat gland, hair, and a hair follicle. Examples of the cells constituting the further tissue ancillary organ include vascular endothelial cells, immune cells other than dendritic cells, melanocytes, hair follicle cells, hair papilla cells, sebaceous gland cells, and fat cells. In one or more embodiments, the artificial skin model of the present disclosure may include cells other than the above cells. Examples of the other cells include cancer cells and cells that are present or can be present around cancer.

The "coated cells" in the present specification include cells and a coating film containing an extracellular matrix component, in which the surface of each of the cells is coated with the coating film. The cells to be coated are not particularly limited. In one or more embodiments, the cells to be coated may be adherent cells such as fibroblasts, epithelial cells, vascular endothelial cells, lymphatic endothelial cells, nerve cells, tissue stem cells, embryonic stem cells, and immune cells. The cells may be derived from human cells or cells other than human cells. The cells may be either one type or two or more types. In one or more embodiments, the coated cells can be prepared by a method disclosed in JP 2012-115254 A.

The "dermal tissue layer" in the present specification is the assembly of cells and an extracellular matrix component, in which the cells are layered three-dimensionally. The dermal tissue layer preferably is similar to and/or imitates the form and/or environment of the dermis of the skin. The dermal tissue layer may include the tissue ancillary organ and/or the cells constituting the tissue ancillary organ, as described above.

### [Method for manufacturing artificial skin model]

The method for manufacturing an artificial skin model of the present disclosure includes the following: preparing coated cells in which a cell surface is coated with a coating film containing an extracellular matrix component forming a dermal tissue layer by culturing coated cells, so that the coated cells are layered three-dimensionally; forming a basal layer including type IV collagen on the dermal issue layer by bringing type IV collagen into contact with the dermal tissue layer; and forming an epidermal layer by arranging epidermal cells on the basal layer, wherein at least one of the dermal tissue layer and the epidermal tissue layer includes dendritic cells.

The method for manufacturing an artificial skin model of the present disclosure can provide an artificial skin model that includes a dermal tissue layer and an epidermal layer, and that can measure a transepithelial electric resistance (TER). Therefore, the use of the artificial skin model of the present disclosure can evaluate, e.g., the barrier function of the skin (epidermal layer) in the state closer to in vivo. The mechanism capable of measuring the TER in the artificial skin model manufactured by the manufacturing method of the present disclosure is not clear, but can be estimated as follows. When the differentiation of epidermal cells is induced on the dermal tissue layer formed by culturing the coated cells, the denseness of the epidermal layer can be improved, and the adjacent epidermal cells can adhere closely together, so that tight junctions can be formed between the epidermal cells. However, the present disclosure should not be limited to this mechanism.

In the skin, the tight junctions formed between the cells of the epidermal layer function as a physical barrier that restricts the movement of substances, and the Langerhans cells function as an immunological barrier like a sensor. These functions are effective in controlling the invasion of foreign substances such as pathogenic bacteria and stimulants from the outside. In one or more embodiments, the artificial skin model manufactured by the manufacturing method of the present disclosure includes not only the tight junctions formed between the cells of the epidermal layer, but also the dendritic cells present in the epidermal layer and/or the dermal tissue layer. With this artificial skin model, the drug evaluation and/or the immune response evaluation can be performed in the state closer to the actual skin.

### [Dermal tissue layer]

The dermal tissue layer is formed by culturing coated cells in which a cell surface is coated with a coating film containing an extracellular matrix component. When the coated cells are cultured, the coated cells are accumulated and the adjacent coated cells adhere to each other via the coating film containing an extracellular matrix component. Thus, the coated cells are layered three-dimensionally to form a dermal tissue layer.

The coated cells include cells and a coating film that contains an extracellular matrix component and covers each of the cells. The "extracellular matrix component" in the present specification is a biological substance that is filled into a space outside of the cells in a living body and has the functions of serving as a framework, providing a scaffold, and/or holding biological factors. The extracellular matrix component may further include a substance that can have the functions of serving as a framework, providing a scaffold, and/or holding biological factors in in vitro cell culture.

The coating film containing an extracellular matrix component preferably includes a film containing a material A and a film containing a material B that interacts with the material A. In one or more embodiments, the combination of the material A and the material B may be (i) a combination of a protein or polymer having an RGD sequence (also referred to as a "material having an RGD sequence" in the following) and a protein or polymer that interacts with the protein or polymer having an RGD sequence (also referred to as an "interacting material" in the following) or (ii) a combination of a protein or polymer having a positive charge (also referred to as a "material having a positive charge" in the following) and a protein or polymer having a negative charge (also referred to as a "material having a negative charge" in the following).

In one or more embodiments, the thickness of the coating film containing an extracellular matrix component is preferably 1 nm to 1 × 10³ nm or 2 nm to 1 × 10² nm, and more preferably 3 nm to 1 × 10² nm because this can provide a dermal tissue layer in which the coated cells are more densely layered. The thickness of the coating film containing an extracellular matrix component can be appropriately controlled, e.g., by the number of films constituting the coating film. The coating film containing an extracellular matrix component is not particularly limited and may be either a single layer or a multi-layer such as 3, 5, 7, 9, 11, 13, 15 layers or more. The thickness of the coating film may be determined by a method as described in the examples.

The coated cells are cultured, e.g., by seeding the coated cells on a substrate and incubating the coated cells for a predetermined time. As a result of the incubation, the adjacent coated cells adhere to each other via the coating film containing an extracellular matrix component, and the coated cells are layered three-dimensionally. Moreover, since the adjacent coated cells are densely arranged, a dermal tissue layer with a compact structure can be formed. The incubation conditions are not particularly limited and may be appropriately determined in accordance with the cells. In one or more embodiments, the incubation temperature may be 4 to 60°C, 20 to 40°C, or 30 to 37°C. In one or more embodiments, the incubation time may be 1 to 168 hours, 3 to 24 hours, or 3 to 12 hours. The culture medium is not particularly limited and may be appropriately determined in accordance with the cells. Examples of the culture medium include Eagle's MEM medium, Dulbecco's Modified Eagle medium (DMEM), Modified Eagle medium (MEM), Minimum Essential medium, RDMI, GlutaMAX medium, and serum-free medium.

The density of the coated cells during seeding may be appropriately determined, e.g., by the number of cell layers included in the dermal tissue layer to be formed. In one or more embodiments, the density may be 1 × 10² cells/cm³ to 1 × 10⁹ cells/cm³, 1 × 10⁴ cells/cm³ to 1 × 10⁸ cells/cm³, or 1 × 10⁵ cells/cm³ to 1 × 10⁷ cells/cm³.

For ease of preparation and handling of air-liquid culture to induce the differentiation of epidermal cells, it is preferable that the coated cells are cultured on a membrane filter. More preferably, the coated cells are cultured using a culture plate that includes a membrane filter. Further preferably, the coated cells are cultured using a culture plate that includes a housing portion and a base portion, in which the base portion serves as a membrane filter. The housing portion is preferably transparent. These culture plates may be commercial products. The commercial products include Transwell (registered trademark), Cell Culture Insert (trade name), etc.

The pore size of the membrane filter is not particularly limited as long as the cultured cells can remain on the membrane filter. In one or more embodiments, the pore size may be 0.1 µm to 2 µm or 0.4 µm to 1.0 µm. The material of the membrane filter may be, e.g., polyethylene terephthalate (PET), polycarbonate, or polytetrafluoroethylene (PTFE).

The dermal tissue layer thus formed can maintain the laminated structure of the cells and have excellent long-term stability even if the dermal tissue layer is stored, e.g., for 2 weeks or more, preferably 3 weeks or more, more preferably 4 weeks or more, even more preferably 5 weeks or more, and further preferably 6 weeks or more after the formation of the dermal tissue layer. The dermal tissue layer may be formed once or formed repeatedly more than once. By forming the dermal tissue layer repeatedly, a multi-layered dermal tissue layer can be obtained, in which more cells are layered.

The thickness of the dermal tissue layer and the number of cells to be layered (i.e., the number of layers) in the dermal tissue layer are not particularly limited. In one or more embodiments, the number of cells to be layered (i.e., the number of layers) in the dermal tissue layer is preferably 3 layers or more, 5 layers or more, 6 layers or more, 10 layers or more, or 15 layers or more because this allows the dermal tissue layer to have properties and/or functions similar to those of the biological tissue of human or the like. The upper limit of the number of cells to be layered is not particularly limited. In one or more embodiments, the upper limit may be 100 layers or less, 50 layers or less, 40 layers or less, 30 layers or less, or 20 layers or less.

In the method for manufacturing an artificial skin model of the present disclosure, in one or more embodiments, the formation of the dermal tissue layer may include culturing the coated cells and the dendritic cells in which a cell surface is coated with a coating film containing an extracellular matrix component.

### [Basal layer]

The basal layer is prepared by bringing collagen IV into contact with the dermal tissue layer. In one or more embodiments, the contact between collagen IV and the dermal tissue layer can be made, e.g., by applying a solution containing collagen IV to the dermal tissue layer, immersing the dermal tissue layer in the solution containing collagen IV, or dropping or spraying the solution containing collagen IV on the dermal tissue layer.

The solution containing collagen IV may contain at least collagen IV In one or more embodiments, the solution may contain collagen IV and a solvent or a dispersion medium (also referred to as a "solvent" in the following). In one or more embodiments, the content of collagen IV in the solution containing collagen IV is preferably 0.0001 to 1 mass%, 0.01 to 0.5 mass%, or 0.02 to 0.1 mass%. In one or more embodiments, the solvent may be, e.g., an aqueous solvent such as water, phosphate buffered saline (PBS), or buffer. Examples of the buffer include the following: Tris buffer such as Tris-HCl buffer; phosphate buffer; HEPES buffer; citrate-phosphate buffer; glycylglycine-sodium hydroxide buffer; Britton-Robinson buffer; and GTA buffer. The pH of the solvent is not particularly limited. In one or more embodiments, the pH is preferably 3 to 11, 6 to 8, or 7.2 to 7.4.

In one or more embodiments, the basal layer may include laminin in addition to collagen IV. When the basal layer includes collagen IV and laminin, in one or more embodiments, it is preferable that the dermal tissue layer is brought into contact with collagen IV and laminin alternately.

### [Epidermal layer]

The epidermal layer can be formed by culturing epidermal cells on the basal layer and then subjecting the epidermal cells to air-liquid culture to induce the differentiation of the epidermal cells. The epidermal cells may be, e.g., epidermal keratinocytes.

It is preferable that the epidermal cells are cultured, e.g., by seeding the epidermal cells on the basal layer and incubating the epidermal cells for a predetermined time. The surface of the epidermal cells may be either coated or not coated with a coating film containing an extracellular matrix component. For ease of operation, the epidermal cells in which the cell surface is not coated with a coating film containing an extracellular matrix component are preferably used. In this case, it is preferable that the surface of the dermal tissue layer has been previously coated with a coating film containing an extracellular matrix component by a method disclosed in JP 2012-115254 A so that the epidermal cells adhere to the dermal tissue layer. The incubation conditions are not particularly limited and may be appropriately determined in accordance with the cells. In one or more embodiments, the incubation temperature may be 4 to 60°C, 20 to 40°C, or 30 to 37°C. In one or more embodiments, the incubation time may be 1 to 3 days or 1 to 2 days.

The culture medium is not particularly limited and may be appropriately determined in accordance with the cells. The preferred culture medium is a culture medium used for the growth of the epidermal cells. The culture medium used for the growth of the epidermal cells may be, e.g., a serum-free medium. Examples of the serum-free medium include the following: MCDB 153 medium; EpiLife (registered trademark) medium; medium obtained by modifying the amino acid composition or the like of the MCDB 153 medium or the EpiLife medium; and medium obtained by mixing Dulbecco's Modified Eagle medium (DMEM) and Ham's F-12 medium in a predetermined ratio. The medium obtained by modifying the amino acid composition of the MCDB 153 medium may be, e.g., MCDB 153 modified medium (e.g., see JP 2005-269923 A). In the MCDB 153 modified medium, the ratio of the amino acids in the MCDB 153 medium is modified by multiplying: L-aspartic acid (salt) by 1.5 to 4 times; L-isoleucine (salt) by 22 to 26 times; L-glutamine (salt) by 1.5 to 3 times; L-glutamic acid (salt) by 1.1 to 2 times; L-tyrosine (salt) by 3 to 6 times; L-tryptophan (salt) by 5 to 7 times; L-valine (salt) by 0.4 to 0.7 times; L-histidine (salt) by 2 to 4 times; L-proline (salt) by 0.4 to 0.7 times; L-phenylalanine (salt) by 4 to 7 times; L-methionine (salt) by 4 to 6 times; and L-lysine (salt) by 1.1 to 2 times, and the content of L-glutamine (salt) in the total of the amino acids is set to 65 wt% or more. Table 1 shows the amino acid composition of the MCDB 153 medium.

| (Table 1) | |
|---|---|
| Amino acid | Ratio (mg/L) |
| L-arginine hydrochloride | 210.67 |
| L-asparagine (monohydrate) | 15 |
| L-aspartic acid | 3.99 |
| L-cysteine hydrochloride | 37.83 |
| L-glutamic acid | 14.7 |
| L-glutamine | 876 |
| glycine | 7.51 |
| L- histidine | 12.42 |
| L-isoleucine | 1.97 |
| L-leucine | 65.6 |
| L-lysine hydrochloride | 18.27 |
| L-methionine | 4.48 |
| L-phenylalanine | 4.95 |
| L-proline | 34.54 |
| L-serine | 63.05 |
| L-threonine | 11.9 |
| L-tryptophane | 3.06 |
| L-tyrosine | 2.72 |
| L-valine | 35.15 |

The culture medium may include, e.g., salts or vitamins. Examples of the salts include potassium chloride, sodium chloride, magnesium chloride, and dibasic sodium phosphate. Examples of the vitamins include choline chloride, cyanocobalamin, nicotinamide, D-pantothenic acid or its salt, pyridoxine hydrochloride or pyridoxal hydrochloride, D-biotin, thiamin hydrochloride, riboflavin, folic acid, DL-α-lipoic acid, and myo-inositol.

In one or more embodiments, the density of the epidermal cells during seeding may be 1 × 10² cells/cm² to 1 × 10⁹ cells/cm², 1 × 10⁴ cells/cm² to 1 × 10⁸ cells/cm², or 1 × 10⁵ cells/cm² to 1 × 10⁷ cells/cm².

The air-liquid culture is performed in such a manner that the culture medium is replaced with a keratinized medium, and then the epidermal cells are incubated with their surfaces being exposed to the air. The incubation temperature is, e.g., 4 to 60°C, preferably 20 to 40°C, and more preferably 30 to 37°C. The incubation time is, e.g., 1 to 40 days, preferably 5 to 30 days, and more preferably 7 to 10 days. The keratinized medium (multi-layered medium) may be a culture medium obtained, e.g., by adding calcium and/or fetal bovine serum to the culture medium that has been used for the growth of the epidermal cells. The calcium concentration of the culture medium is preferably about 0.4 mM to 2.0 mM.

### [Dendritic cells]

In the method for manufacturing an artificial skin model of the present disclosure, the dermal tissue layer and/or the epidermal layer include dendritic cells. In one or more embodiments, when the dermal tissue layer is formed, the dendritic cells may be mixed with the coated cells (e.g., fibroblasts) for forming the dermal tissue layer and then cultured, or the dendritic cells may be arranged between the cell layers of the coated cells for forming the dermal tissue layer and then cultured. When the epidermal layer is formed, the dendritic cells may be mixed with the epidermal cells and then cultured, or the dendritic cells may be arranged between the cell layers of the epidermal cells and then cultured. In one or more embodiments, the surface of the dendritic cells may be coated with a coating film containing an extracellular matrix component.

The density of the dendritic cells during seeding may be appropriately determined, e.g., by the number of cell layers included in the dermal tissue layer and/or the epidermal layer. When the dendritic cells are arranged in the dermal tissue layer, in one or more embodiments, the density of the dendritic cells may be 3 to 200000 cells/cm², 20000 to 100000 cells/cm², or 40000 to 80000 cells/cm². When the dendritic cells are arranged by mixing with the coated cells for forming the dermal tissue layer, in one or more embodiments, the ratio of the dendritic cells to the coated cells (dendritic cells : coated cells) may be 1 : 99 to 99 : 1, 10 : 90 to 90 : 10, or 10 : 90 to 50 : 50. When the dendritic cells are arranged by mixing with the coated cells for forming the dermal tissue layer, the dendritic cells can be cultured under the same conditions as the formation of the dermal tissue layer. When the dendritic cells are arranged in the epidermal layer, in one or more embodiments, the density of the dendritic cells may be 3 to 200000 cells/cm², 20000 to 100000 cells/cm², or 40000 to 80000 cells/cm². When the dendritic cells are arranged by mixing with the epidermal cells, in one or more embodiments, the ratio of the dendritic cells to the epidermal cells (dendritic cells : epidermal cells) may be 1 : 99 to 99 : 1, 10 : 90 to 90 : 10, or 10 : 90 to 50: 50.

In one or more embodiments, the method for manufacturing an artificial skin model of the present disclosure may include forming a tissue ancillary organ in the dermal tissue layer. The tissue ancillary organ can be formed in the following manner. For example, when the dermal tissue layer is formed, cells for forming the tissue ancillary organ may be mixed with the coated cells (e.g., fibroblasts) for forming the dermal tissue layer and then cultured, or cells for forming the tissue ancillary organ may be arranged between the cell layers of the coated cells for forming the dermal tissue layer and then cultured. The cells for forming the tissue ancillary organ may be arranged between the cell layers in the following manner. For example, the cells for forming the tissue ancillary organ are arranged on the cell layer of the coated cells and cultured for a predetermined time as needed. Subsequently, the coated cells are arranged on the cells for forming the tissue ancillary organ. Therefore, the method for manufacturing an artificial skin model of the present disclosure may include the following steps in the formation of the dermal tissue layer: (i) culturing coated cells to form a cell layer in which the coated cells are layered; (ii) arranging cells for forming the tissue ancillary organ on the cell layer, (iii) arranging coated cells on the cell layer on which the cells for forming the tissue ancillary organ have been arranged, and (iv) culturing the coated cells to form a cell layer in which the coated cells are layered.

If the tissue ancillary organ is an organ having a net-like structure in the skin such as blood vessels or lymphatic vessels, it is preferable that the cells for forming the tissue ancillary organ are cultured while they are sandwiched between the cell layers of the coated cells. Thus, since the cells for forming the tissue ancillary organ are sandwiched between the cell layers and cultured, a dense blood vessel network or lymphatic vessel network closer to that in a living body of human can be formed. When blood vessels are formed as the tissue ancillary organ, the cells for forming the tissue ancillary organ may be vascular endothelial cells. When lymphatic vessels are formed as the tissue ancillary organ, the cells for forming the tissue ancillary organ may be lymphatic endothelial cells.

The cells for forming the tissue ancillary organ are as described above. Stem cells and/or precursor cells of the cells for forming the tissue ancillary organ may be used in addition to or instead of the cells for forming the tissue ancillary organ. Similarly to the coated cells for forming the dermal tissue layer, the surface of the cells for forming the tissue ancillary organ may be coated with a coating film containing an extracellular matrix component. Alternatively, like the formation of the epidermal layer, the cells for forming the tissue ancillary organ may be used without any coating film. In terms of improving the working efficiency, it is preferable that a coating film containing an extracellular matrix component is formed on the surface of the cells for forming the tissue ancillary organ. The number of cells to be seeded, the culture conditions, or the like may be appropriately determined, e.g., by the cells for forming the tissue ancillary organ. The coating film containing an extracellular matrix component can be formed in the same manner as the coated cells.

The method for manufacturing an artificial skin model of the present disclosure includes preparing coated cells. The coated cells can be prepared by bringing cells into contact with an extracellular matrix component. In one or more embodiments, it is preferable that the coated cells are prepared by bringing cells into contact with a material A and a material B alternately because this can improve the adhesiveness between the adjacent coated cells, and thus can provide a dermal tissue layer in which the coated cells are more densely layered. As described above, the combination of the material A and the material B may be a combination of the material having an RGD sequence and the interacting material or a combination of the material having a positive charge and the material having a negative charge.

### (Material having RGD sequence)

The material having an RGD sequence is a protein or polymer having an "Arg-Gly-Asp" (RGD) sequence, which is an amino acid sequence that is associated with cell adhesion activity. The material "having an RGD sequence" in the present specification may be a material that inherently has an RGD sequence or a material to which an RGD sequence is chemically bound. The material having an RGD sequence is preferably biodegradable.

The protein having an RGD sequence may be, e.g., a conventionally known adhesive protein or a water-soluble protein having an RGD sequence. Examples of the adhesive protein include fibronectin, vitronectin, laminin, cadherin, and collagen. Examples of the water-soluble protein having an RGD sequence include collagen, gelatin, albumin, globulin, proteoglycan, enzymes, and antibodies, to each of which an RGD sequence is bound.

The polymer having an RGD sequence may be, e.g., a naturally occurring polymer or a synthetic polymer. Examples of the naturally occurring polymer having an RGD sequence include water-soluble polypeptide, low molecular weight peptide, polyamino acid such as α-polylysine or ε-polylysine, and sugar such as chitin or chitosan. Examples of the synthetic polymer having an RGD sequence include straight-chain, graft, comb, dendritic, or star polymers or copolymers having an RGD sequence. Examples of the polymers or copolymers include the following: polyurethane, polycarbonate, polyamide, or copolymers thereof polyester; poly(N-isopropylacrylamide-co-polyacrylic acid); polyamidoamine dendrimer; polyethylene oxide; poly(ε-caprolactam); polyacrylamide; and poly(methyl methacrylate-γ-polyoxyethylene methacrylate).

Among them, the material having an RGD sequence is preferably fibronectin, vitronectin, laminin, cadherin, polylysine, elastin, collagen to which an RGD sequence is bound, gelatin to which an RGD sequence is bound, chitin, or chitosan. The material having an RGD sequence is more preferably fibronectin, vitronectin, laminin, polylysine, collagen to which an RGD sequence is bound, or gelatin to which an RGD sequence is bound.

### (Interacting material)

The interacting material is a protein or polymer that interacts with the material having an RGD sequence. The term "interact" in the present specification means that the material having an RGD sequence and the interacting material approach each other to the extent that bonding, adhesion, adsorption, or electron transfer can occur chemically and/or physically between them, e.g., due to electrostatic interaction, hydrophobic interaction, hydrogen bond, charge transfer interaction, covalent bond formation, specific interaction between proteins, and/or Van der Waals force. The interacting material is preferably biodegradable.

The protein that interacts with the material having an RGD sequence may be, e.g., collagen, gelatin, proteoglycan, integrin, enzymes, or antibodies. The polymer that interacts with the material having an RGD sequence may be, e.g., a naturally occurring polymer or a synthetic polymer. Examples of the naturally occurring polymer that interacts with the material having an RGD sequence include water-soluble polypeptide, low molecular weight peptide, polyamino acid, elastin, sugar such as heparin, heparan sulfate, or dextran sulfate, and hyaluronic acid. Examples of the polyamino acid include polylysine such as α-polylysine or ε-polylysine, polyglutamic acid, and polyaspartic acid. Examples of the synthetic polymer that interacts with the material having an RGD sequence include straight-chain, graft, comb, dendritic, or star polymers or copolymers having an RGD sequence. Examples of the polymers or copolymers include the following: polyurethane, polyamide, polycarbonate, or copolymers thereof polyester; polyacrylic acid; polymethacrylic acid; polyethylene glycol-graft-polyacrylic acid; poly(N-isopropylacrylamide-co-polyacrylic acid); polyamidoamine dendrimer; polyethylene oxide; poly(ε-caprolactam); polyacrylamide; and poly(methyl methacrylate-γ-polyoxyethylene methacrylate).

Among them, the interacting material is preferably gelatin, dextran sulfate, heparin, hyaluronic acid, globulin, albumin, polyglutamic acid, collagen, or elastin. The interacting material is more preferably gelatin, dextran sulfate, heparin, hyaluronic acid, or collagen. The interacting material is further preferably gelatin, dextran sulfate, heparin, or hyaluronic acid.

The combination of the material having an RGD sequence and the interacting material is not particularly limited and may be a combination of different materials that interact with each other. Specifically, one of the materials may be a polymer or protein having an RGD sequence, and the other may be a polymer or protein that reacts with the polymer or protein having an RGD sequence. Examples of the combination of the material having an RGD sequence and the interacting material include the following: fibronectin and gelatin; fibronectin and ε-polylysine; fibronectin and hyaluronic acid; fibronectin and dextran sulfate; fibronectin and heparin; fibronectin and collagen; laminin and gelatin; laminin and collagen; polylysine and elastin; vitronectin and collagen; and RGD-bound collagen or RGD-bound gelatin and collagen or gelatin. Among them, the combination is preferably fibronectin and gelatin, fibronectin and ε-polylysine, fibronectin and hyaluronic acid, fibronectin and dextran sulfate, fibronectin and heparin, or laminin and gelatin. The combination is more preferably fibronectin and gelatin. Each of the material having an RGD sequence and the interacting material may be either one type or two or more types as long as they interact with each other.

### (Material having positive charge)

The material having a positive charge is a protein or polymer having a positive charge. The protein having a positive charge is preferably a water-soluble protein. Examples of the water-soluble protein include basic collagen, basic gelatin, lysozyme, cytochrome c, peroxidase, and myoglobin. The polymer having a positive charge may be, e.g., a naturally occurring polymer or a synthetic polymer. Examples of the naturally occurring polymer include water-soluble polypeptide, low molecular weight peptide, polyamino acid, and sugar such as chitin or chitosan. Examples of the polyamino acid include polylysine such as poly(α-lysine) or poly(ε-lysine), polyarginine, and polyhistidine. Examples of the synthetic polymer include straight-chain, graft, comb, dendritic, or star polymers or copolymers. Examples of the polymers or copolymers include the following: polyurethane, polyamide, polycarbonate, or copolymers thereof polyester; polydiallyldimethylammonium chloride (PDDA); polyallylamine hydrochloride; polyethyleneimine; polyvinylamine; and polyamidoamine dendrimer.

### (Material having negative charge)

The material having a negative charge is a protein or polymer having a negative charge. The protein having a negative charge is preferably a water-soluble protein. Examples of the water-soluble protein include acid collagen, acid gelatin, albumin, globulin, catalase, β-lactoglobulin, thyroglobulin, α-lactalbumin, and ovalbumin. The polymer having a negative charge may be, e.g., a naturally occurring polymer or a synthetic polymer. Examples of the naturally occurring polymer include water-soluble polypeptide, low molecular weight peptide, polyamino acid such as poly(β-lysine), and dextran sulfate. Examples of the synthetic polymer include straight-chain, graft, comb, dendritic, or star polymers or copolymers. Examples of the polymers or copolymers include the following: polyurethane, polyamide, polycarbonate, or copolymers thereof polyester; polyacrylic acid; polymethacrylic acid; polystyrene sulfonic acid; polyacrylamide methylpropane sulfonic acid; carboxy-terminated polyethylene glycol; polydiallyldimethylammonium salt; polyallylamine salt; polyethyleneimine; polyvinylamine; and polyamidoamine dendrimer.

Examples of the combination of the material having a positive charge and the material having a negative charge include the following: ε-polylysine salt and polysulfonate; ε-polylysine and polysulfonate; chitosan and dextran sulfate; polyallylamine hydrochloride and polystyrene sulfonate; polydiallyldimethylammonium chloride and polystyrene sulfonate; and polydiallyldimethylammonium chloride and polyacrylate. The combination is preferably ε-polylysine salt and polysulfonate or polydiallyldimethylammonium chloride and polyacrylate. The polysulfonate may be, e.g., poly(sodium sulfonate) (PSS). Each of the material having a positive charge and the material having a negative charge may be either one type or two or more types as long as they interact with each other.

Hereinafter, a method for preparing coated cells will be described. In this method, first, cells are brought into contact with a solution A containing a material having an RGD sequence, and then the cells are brought into contact with a solution B containing a material that interacts with the material having an RGD sequence, thereby preparing coated cells.

First, cells are brought into contact with the solution A. Consequently, a film containing the material having an RGD sequence is formed on the surface of each of the cells, and thus the surface of each of the cells is coated with the film containing the material having an RGD sequence. The contact between the cells and the solution A can be made, e.g., by applying or adding the solution A to the cells, immersing the cells in the solution A, or dropping or spraying the solution A on the cells. In particular, for ease of operation, it is preferable that the cells are brought into contact with the solution A by immersion in the solution A.

In one or more embodiments, the contact conditions may be appropriately determined, e.g., by the contact process, the type of the material having an RGD sequence and/or the type of cells, and the concentration of the solution. In one or more embodiments, the contact time is preferably 30 seconds to 24 hours, 1 minute to 60 minutes, 1 minute to 15 minutes, 1 minute to 10 minutes, or 1 minute to 5 minutes. In one or more embodiments, the ambient temperature and/or the temperature of the solution during contact is preferably 4 to 60°C, 20 to 40°C, or 30 to 37°C.

The solution A may contain at least the material having an RGD sequence, and preferably contains the material having an RGD sequence and a solvent or a dispersion medium (also simply referred to as a "solvent" in the following). In one or more embodiments, the content of the material having an RGD sequence in the solution A is preferably 0.0001 to 1 mass%, 0.01 to 0.5 mass%, or 0.02 to 0.1 mass%. In one or more embodiments, the solvent may be, e.g., an aqueous solvent such as water, phosphate buffered saline (PBS), or buffer. Examples of the buffer include the following: Tris buffer such as Tris-HCl buffer; phosphate buffer; HEPES buffer; citrate-phosphate buffer; glycylglycine-sodium hydroxide buffer; Britton-Robinson buffer; and GTA buffer. The pH of the solvent is not particularly limited. In one or more embodiments, the pH is preferably 3 to 11, 6 to 8, or 7.2 to 7.4.

In one or more embodiments, the solution A may further contain salt, a cell growth factor, cytokine, chemokine, hormone, biologically active peptide, or a pharmaceutical composition. Examples of the pharmaceutical composition include a therapeutic agent for diseases, a preventive, an inhibitor, an antibacterial agent, and an anti-inflammatory agent. Examples of the salt include sodium chloride, calcium chloride, sodium hydrogencarbonate, sodium acetate, sodium citrate, potassium chloride, dibasic sodium phosphate, magnesium sulfate, and sodium succinate. The salt may be either one type or two or more types. Both the solution A and the solution B may contain the salt, or one of them may contain the salt. The salt concentration in the solution A is not particularly limited and may be, e.g., 1 × 10⁻⁶ M to 2 M, preferably 1 × 10⁻⁴ M to 1 M, and more preferably 1 × 10⁻⁴ M to 0.05 M.

Next, the material that has not been used for the formation of the film containing the material having an RGD sequence is separated. The separation may be performed, e.g., by centrifugation or filtration. When the material is separated by centrifugation, e.g., the solution A in which the cells are dispersed is centrifuged, and then the supernatant is removed. The centrifugation conditions may be appropriately determined, e.g., by the type of cells, the concentration of cells, and the composition of the materials contained in the solution A.

In addition to the above separation, a washing operation is preferably performed. The washing operation may be performed, e.g., by centrifugation or filtration. When the washing operation is performed by centrifugation, e.g., a solvent is added to the cells from which the supernatant has been removed, and this solution is centrifuged so that the supernatant is removed. It is preferable that the solvent used for washing is the same as that of the solution A.

Next, the cells coated with the film containing the material having an RGD sequence are brought into contact with the solution B. Consequently, a film containing the interacting material is formed on the surface of the film containing the material having an RGD sequence, and thus the surface of each of the cells, which has been coated with the film containing the material having an RGD sequence, is further coated with the film containing the interacting material. The contact between the cells and the solution B can be made in the same manner as the contact between the cells and the solution A except that the interacting material is used instead of the material having an RGD sequence.

By repeatedly bringing the cells into contact with the solution A and the solution B alternately, the film containing the material having an RGD sequence and the film containing the interacting material can be alternately laminated to form a coating film containing an extracellular matrix component on the entire surface of each of the cells. The number of times of the contact between the cells and the solution A or the solution B may be appropriately determined, e.g., by the thickness of the coating film containing an extracellular matrix component to be formed.

### [Artificial skin model]

In one or more embodiments, the present disclosure relates to an artificial skin model manufactured by the manufacturing method of the present disclosure, wherein the artificial skin model includes the following: a dermal tissue layer that includes an extracellular matrix component and layered cells; a basal layer that includes type IV collagen and is formed on the dermal tissue layer; and an epidermal layer that is formed on the basal layer, wherein the dermal tissue layer and optionally the epidermal layer includes dendritic cells, wherein cells in the dermal tissue layer are layered via the extracellular matrix component, and wherein the dermal tissue layer includes lymphatic vessels. With the artificial skin model of the present disclosure, e.g., the drug effect test, immune response, pharmacological test, and safety test of a test substance can be evaluated in the environment closer to the actual skin. Moreover, the artificial skin model of the present disclosure can also be used as a covering material for treating burn, wound, etc.

In the epidermal layer, e.g., it is preferable that tight junctions are formed between the adjacent epidermal cells. When the tight junctions are formed in the epidermal layer, e.g., the barrier function or the like of the epidermal layer can be evaluated by measuring a TER.

The artificial skin model of the present disclosure includes a tissue ancillary organ formed in the dermal tissue layer. This allows the drug effect test, pharmacological test, and/or safety test of a test substance to be evaluated in the environment closer to the actual skin.

The number of cells to be layered in the dermal tissue layer is not particularly limited and may be 3 layers or more, 5 layers or more, 6 layers or more, or 10 layers or more because this makes it possible for the dermal tissue layer to have properties and functions similar to those of the skin of human or the like. The upper limit of the number of cells to be layered is not particularly limited and may be, e.g., 100 layers or less, 50 layers or less, 40 layers or less, 30 layers or less, or 20 layers or less.

### [Evaluation method]

A method is disclosed herein for evaluating the skin irritation of a test substance with the use of the artificial skin model of the present disclosure. The evaluation method enables the test substance to be evaluated in the environment closer to the actual skin compared to the conventional method. Moreover, the evaluation method can be a very useful tool, e.g., in evaluating the pharmacokinetics of drugs with different molecular weights for the creation (screening) of new drugs, or in evaluating the development of cosmetics, quasi drugs, or the like.

The evaluation method can be performed, e.g., by bringing a test substance into contact with the artificial skin model and measuring an immune response due to the contact with the test substance. The response can be measured, e.g., by measuring a TER. The test substance is a substance to be evaluated. Examples of the test substance include an inorganic compound and an organic compound.

### [Evaluation kit]

An evaluation kit for evaluating the irritation of a test substance is disclosed herein. The evaluation kit includes the artificial skin model of the present disclosure. The evaluation kit enables the evaluation method of the present disclosure to be performed more easily.

The evaluation kit may further include a product that includes at least one of a reagent, a material, a tool, and a device used for a predetermined test and an instruction manual for the evaluation of the test.

### [Artificial skin model manufacturing kit]

An artificial skin model manufacturing kit is disclosed herein. The artificial skin model manufacturing kit preferably includes, e.g., a reagent used for the formation of a coating film containing an extracellular matrix component, and an instruction manual that tells the method for manufacturing an artificial skin model of the present disclosure. The artificial skin model manufacturing kit enables the artificial skin model of the present disclosure to be manufactured more easily.

The artificial skin model manufacturing kit may further include a substrate on which a dermal tissue layer is formed. Due to the presence of the substrate provided with the dermal tissue layer, e.g., the artificial skin model of the present disclosure can be manufactured more easily in a short time, and the time required for forming the tissue ancillary organ can also be shortened.

Hereinafter, the present disclosure will be described in more detail by way of examples. However, the present disclosure is not limited to the following examples. Examples

### [Method for measuring thickness of coating film]

The thickness of the coating film formed on the cell surface was determined in the following manner. First, a coating film was separately formed on a substrate. Using a quartz crystal microbalance (QCM) measuring method, the number of processes (steps) performed and the thickness of the coating film thus formed were measured. Based on the results of the measurement, the thickness of the coating film was calculated in accordance with the number of steps performed during the formation of the coating film on the cell surface. The measurement using the QCM measuring method was performed as follows. A QCM sensor was washed with a piranha solution for 1 minute. Subsequently, the QCM sensor was immersed in a Tris-HCl solution (pH = 7.4) containing 0.2 mg/ml fibronectin (also referred to as "FN" in the following) at 37°C for 1 minute. After the QCM sensor was washed with a Tris-HCl solution (pH = 7.4) and air-dried, a frequency shift was measured (Step 1). Next, the QCM sensor was immersed in a Tris-HCl solution (pH = 7.4) containing 0.2 mg/ml gelatin (also referred to as "G" in the following) at 37°C for 1 minute. After the QCM sensor was washed with a Tris-HCl solution (pH = 7.4) and air-dried, a frequency shift was measured (Step 2). By repeating Step 1 and Step 2 alternately, a coating film was formed on the QCM sensor, and the frequency shit was measured. Based on the resultant frequency shift, the number of steps and the thickness of the coating film formed by the steps were obtained.

### (Example 1)

### [Preparation of coated cells]

Human fibroblasts (normal human dermal fibroblasts (NHDF), produced by Cambrex Corporation) were dispersed at a concentration of 1 × 10⁶ cell/ml in a 50 mM Tris-HCl solution (pH = 7.4) containing 0.2 mg/ml fibronectin. The dispersion state was maintained for 1 minute while this solution was gently stirred by inverting the container. Then, the solution was centrifuged at 2500 rpm for 1 minute (FN immersion operation). After the supernatant was removed, a 50 mM Tris-HCl solution (pH = 7.4) was added so that the cells were dispersed. The dispersion state was maintained for 1 minute while this solution was gently stirred by inverting the container. Then, the solution was centrifuged at 2500 rpm for 1 minute (washing operation). After the supernatant was removed, the cells were dispersed in a 50 mM Tris-HCl solution (pH = 7.4) containing 0.2 mg/ml gelatin. The dispersion state was maintained for 1 minute while this solution was gently stirred by inverting the container. Then, the solution was centrifuged at 2500 rpm for 1 minute (G immersion operation). Subsequently, the washing operation was performed. The FN immersion operation, the washing operation, the G immersion operation, and the washing operation were performed in this order. In this case, a set of the FN immersion operation and the washing operation was defined as one step, and another set of the G immersion operation and the washing operation was defined as one step. Finally, a total of 9 steps, i.e., 5 times of the FN immersion operation and 4 times of the G immersion operation were performed, so that NHDF coated cells were prepared (the thickness of the coating layer: 6 nm).

Coated cells were prepared in the same manner as the preparation of the NHDF coated cells except that human peripheral blood monocyte-derived dendritic cells (also referred to as "MoDC" in the following) were used instead of the NHDF. A total of 9 steps, i.e., 5 times of the FN immersion operation and 4 times of the G immersion operation were performed, so that MoDC coated cells were prepared (the thickness of the coating layer: 6 nm).

### [Preparation of skin model]

First, 5 × 10⁵ NHDF coated cells and 5 × 10⁴ MoDC coated cells were mixed and seeded on a membrane filter of Transwell (manufactured by Corning Incorporated; pore size: 0.4 µm, surface area: 0.33 cm²) placed on a 24-well culture plate. Then, Eagle's MEM medium containing 10 wt% fetal bovine serum was added, and the mixed coated cells were cultured at 37°C for 1 day. Consequently, a dermal tissue layer (thickness: 30 µm) including dendritic cells and 6 layers of the NHDF was obtained.

Next, a Tris solution containing 0.2 mg/ml collagen IV was added to the surface of the dermal tissue layer and incubated for 1 hour, so that a basal layer (thickness: 2.4 nm) including collagen IV was formed on the dermal tissue layer. Then, 1.8 × 10⁵ keratinocytes (also referred to as "KC" in the following) were seeded on the surface of the basal layer and cultured at 37°C for 2 days. Subsequently, the culture medium was replaced with a keratinized medium (multi-layered medium), and a cell culture insert was placed so that a membrane filter of the cell culture insert was positioned at the air-liquid interface of the keratinized medium. In this state, air-liquid culture was performed at 37°C for 7 days to induce differentiation, and thus an artificial skin model was prepared.

In the preparation of the above artificial skin model, the MoDC coated cells were colored with a green fluorescent dye (trade name: Cell Tracker™ Green Fluorescent Probe, product code: PA-3011), the NHDF coated cells were colored with a red fluorescent dye (trade name: Cell Tracker™ Orenge Fluorescent Probe, product code: PA-3012), and the KC were colored with a blue fluorescent dye (trade name: Cell Tracker™ Blue).

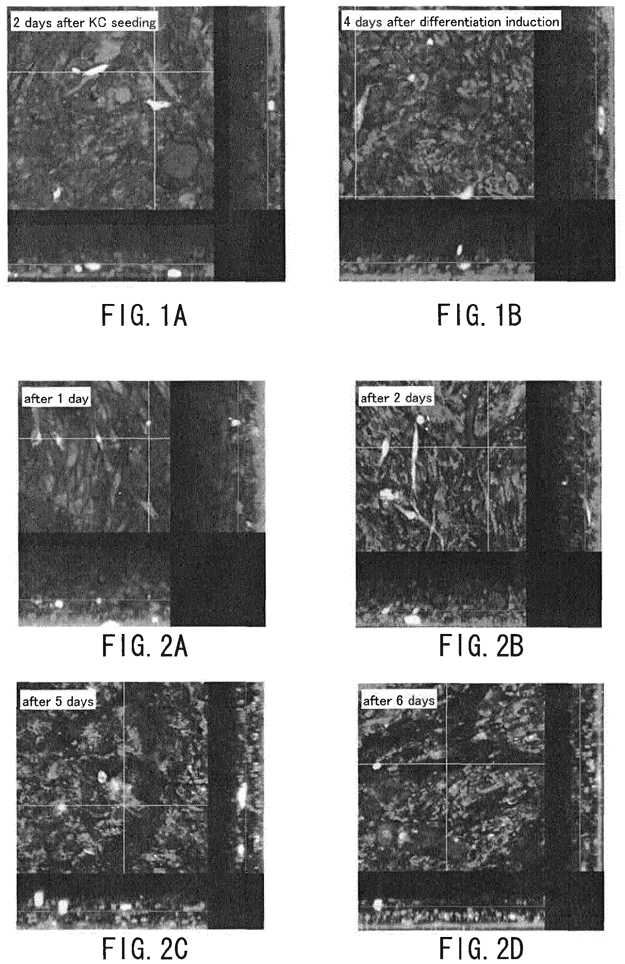

FIG. 1 shows an example of confocal laser scanning microscope (CLSM) images of the prepared artificial skin model (4 days after KC differentiation induction) and a three-dimensional culture construct before differentiation induction (2 days after KC seeding). FIG. 1A is a CLSM image of the artificial skin model. FIG. 1B is a CLSM image of the three-dimensional culture construct before differentiation induction. In FIGS. 1A and 1B, the cells dyed in red are the NHDF, the cells dyed in blue are the KC, and the cells dyed in green are the MoDC.

As shown in FIG. 1A, the method of the present disclosure can provide the artificial skin model in which the dendritic cells are introduced into the dermal layer. The state of adhesion of the dendritic cells is also observed. This indicates that the dendritic cells are alive in the dermal layer.

### (Example 2)

An artificial skin model was prepared in the same manner as Example 1 except that 5 × 10⁴ MoDC coated cells and 5 × 10⁴ HUVEC (human umbilical vein endothelial cells) coated cells were seeded on a first three-dimensional culture layer. First, 2.5 × 10⁵ NHDF coated cells were seeded, and DMEM containing 20% FBS was added. The NHDF coated cells were cultured for 1 day to form a first three-dimensional culture layer (NHDF: 3 layers). Then, 5 × 10⁴ MoDC coated cells and 5 × 10⁴ HUVEC coated cells were seeded on the first three-dimensional culture layer, and DMEM containing 20% FBS was added. The MoDC coated cells and the HUVEC coated cells were cultured for 1 day. Subsequently, 2.5 × 10⁵ NHDF coated cells were seeded, thereby preparing a dermal tissue layer. A basal layer and an epidermal layer were formed in the same procedure as described above. The HUVEC coated cells were prepared in the same procedure as the preparation of the coated cells in Example 1 except that the HUVEC were used instead of the NHDF.

FIG. 2 shows an example of confocal laser scanning microscope (CLSM) images of the prepared artificial skin model. FIG. 2A is an image taken 1 day after KC differentiation induction. FIG. 2B is an image taken 2 days after KC differentiation induction. FIG. 2C is an image taken 5 days after KC differentiation induction. FIG. 2D is an image taken 6 days after KC differentiation induction. In FIGS. 2A to 2D, the cells dyed in red are the NHDF, the cells dyed in blue are the HUVEC, and the cells dyed in green are the MoDC.

As shown in FIG. 2, the method of the present disclosure can provide the artificial skin model in which the dendritic cells and the capillary network are introduced into the dermal layer. Moreover, it is indicated that the dendritic cells are alive even in the coexistence with the capillary network.

### (Reference Example 1)

### [Immune response evaluation]

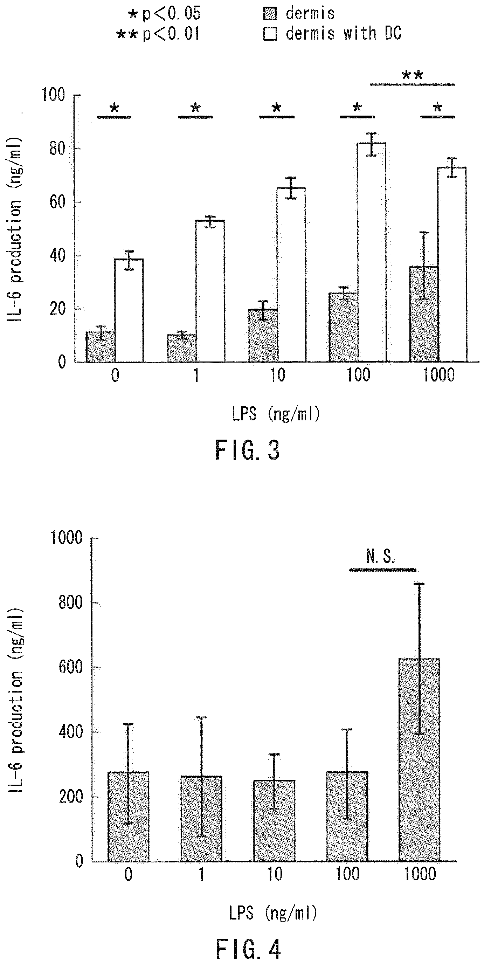

A dermal tissue layer including dendritic cells was prepared in the same manner as Example 1. The culture medium of the dermal tissue layer was changed to DMEM containing 10% FBS (the inside of the insert: 30 µl, the outside of the insert: 1 ml). Lipopolysaccharide (LPS, produced by Sigma-Aldrich Co. LLC.) was added to the inside of the insert and incubated for 24 hours. Then, all the culture medium in the outside of the insert was collected, and interleukin (IL)-6 was quantified by enzyme-linked immunosorbent assay (ELISA (R & D, D6056)). The concentration of the lipopolysaccharide was 0, 1, 10, 100, or 1000 ng/ml. FIG. 3 shows the results.

A dermal tissue layer (without dendritic cells) was prepared in the same manner as Example 1 except that "only the NHDF coated cells" were used instead of "a mixture of the NHDF coated cells and the MoDC coated cells". Using the dermal tissue layer, an immune response was evaluated in the same manner as described above.

FIG. 3 is an example of a graph showing the relationship between the amount of LPS added and the amount of IL-6 produced from the dermal tissue layer. As shown in FIG. 3, the amount of IL-6 produced increases with an increase in the amount of LPS added in both the dermal tissue layer that includes the dendritic cells and the dermal tissue layer that does not include the dendritic cells. Moreover, the amount of IL-6 produced increases significantly in the dermal tissue layer that includes the dendritic cells compared to the dermal tissue layer that does not include the dendritic cells.

### (Example 4)

### [Preparation of skin model]

First, a dermal tissue layer was prepared in the same manner as Example 1 and immersed in a 50 mM Tris-HCl solution (pH = 7.4) containing collagen IV for 20 minutes, so that the dermal tissue layer was coated with collagen IV. Then, 1.8 × 10⁵ KC were seeded and cultured in an adherent culture medium for 2 days. Subsequently, the KC were exposed to the air-liquid interface of a keratinized medium (multi-layered medium) for 7 days to induce differentiation, and thus an artificial skin model was prepared.

Using the artificial skin model, an immune response was evaluated in the same manner as Reference Example 1. FIG. 4 shows the results. FIG. 4 is an example of a graph showing the relationship between the amount of LPS added and the amount of IL-6 produced from the dermal tissue layer. As shown in FIG. 4, in the artificial skin model including the epidermal layer, the amount of IL-6 produced does not change with the amount of LPS added. It can be expected that the artificial skin model of Example 4 has a physical barrier function due to the tight junctions formed between the cells of the epidermal layer, and therefore prevents the permeation of the LPS.

### (Reference Example 2)

NHDF coated cells were seeded and cultured so that the NHDF were arranged in 4 layers. Then, NHDMEC (human dermal microvascular endothelial cells) coated cells were seeded and cultured on top of the NHDF layers so that the NHDMEC were arranged in 1 layer. Moreover, NHDF coated cells were seeded and cultured on the NHDMEC layer so that the NHDF were arranged in 4 layers. These layered cells were cultured in 2.3 ml of DMEM containing 10% FBS for 3 days. Subsequently, 5 × 10⁴ MoDC were seeded and cultured in 2.3 ml of DMEM containing 10% FBS for 1 day. Thus, a dermal tissue layer was formed. The dermal tissue layer was placed in the insert, to which 1 µg/ml LPS was added. The dermal tissue layer was observed by a confocal laser scanning microscope for 30 hours.

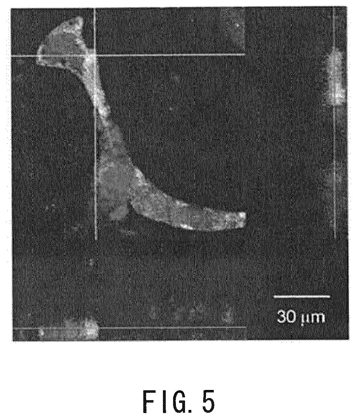

FIG. 5 shows the results. FIG. 5 is an example of a confocal laser scanning microscope (CLSM) image taken 30 hours after the addition of the LPS. As shown in FIG. 5, the state of overlap between the dendritic cells and the NHDMEC after the addition of the LPS is observed. Although the details of this are not clear, it is known that the dendritic cells are transferred to the lymphatic vessels when they are activated by an antigen in a living body. Therefore, the observation indicates that the same mechanism as that in a living body also occurs in the dermal tissue layer of Reference Example 2. Moreover, it has been reported that, in a living body, a CC chemokine receptor 7 is expressed in the activated dendritic cells, while CC chemokine ligands 21, 19, which are ligands of the CC chemokine receptor 7, are produced in the lymphatic vessels. Therefore, in the dermal tissue layer of Reference Example 2, the above cytokine produced in the lymphatic vessels may induce the dendritic cells to be the NHDMEC.

### Industrial Applicability

The present disclosure can provide an artificial skin model that allows permeability of a test substance, a minute response associated with the permeability, or the like to be evaluated. The present disclosure is useful, e.g., in the fields of cosmetics, medicine, and pharmaceutical production.

## Claims

1. A method for manufacturing an artificial skin tissue, comprising:
preparing coated cells in which a cell surface is coated with a coating film containing an extracellular matrix component,
forming a dermal tissue layer by culturing the coated cells, so that the coated cells are layered three-dimensionally ;
forming a basal layer including type IV collagen on the dermal tissue layer by bringing type IV collagen into contact with the dermal tissue layer; and
forming an epidermal layer by arranging epidermal cells on the basal layer,
wherein at least one of the dermal tissue layer and the epidermal layer includes dendritic cells.

2. The method according to claim 1, wherein the formation of the dermal tissue layer comprises culturing the coated cells and dendritic cells in which a cell surface is coated with a coating film containing an extracellular matrix component.

3. The method according to claim 1 or 2, wherein the formation of the epidermal layer comprises arranging epidermal cells and dendritic cells on the basal layer.

4. The method according to any one of claims 1 to 3, wherein the formation of the basal layer comprises bringing type IV collagen and laminin into contact with the dermal tissue layer alternately so that a type IV collagen layer and a laminin layer are alternately formed on the dermal tissue layer.

5. The method according to any one of claims 1 to 4, wherein the coated cells include fibroblasts in which the cell surface is coated with a coating film containing an extracellular matrix component.

6. The method according to claim 1, wherein the formation of the dermal tissue layer comprises mixing dendritic cells and the coated cells, and then culturing the mixed dendritic cells and coated cells.

7. The method according to claim 6, wherein the density of the dendritic cells is 3 to 200000 cells/cm².

8. The method according to any one of claims 1 to 7, wherein the formation of the dermal tissue layer comprises mixing cells for forming the tissue ancillary organ with the coated cells, or arranging cells for forming the tissue ancillary organ between the cell layers of the coated cells.

9. The method according to claim 8, wherein the cell for forming the tissue ancillary organ is vascular endothelial cell and/or lymphatic endothelial cell.

10. The method according to any one of claims 1 to 9, wherein the density of the coated cells is 1 × 10² cells/cm³.

11. An artificial skin tissue manufactured by the method according to any one of claims 1 to 10, wherein said artificial skin tissue comprises:
a dermal tissue layer that includes an extracellular matrix component and layered cells;
a basal layer that includes type IV collagen and is formed on the dermal tissue layer; and
an epidermal layer that is formed on the basal layer,
wherein the dermal tissue layer and optionally the epidermal layer includes dendritic cells,
wherein cells in the dermal tissue layer are layered via the extracellular matrix component, and
wherein the dermal tissue layer includes lymphatic vessels.

12. The artificial skin tissue according to claim 11, wherein the ratio of the dendritic cells to cells other than the dendritic cells for forming the dermal tissue layer is 1 : 99 to 50 : 50 in the dermal tissue layer.

## Patentansprüche

1. Verfahren zum Herstellen eines künstlichen Hautgewebes, umfassend:
Aufbereiten beschichteter Zellen, wobei eine Zelloberfläche mit einem Beschichtungsfilm beschichtet ist, der eine extrazelluläre Matrixkomponente enthält,
Bilden einer Hautgewebeschicht durch Kultivieren der beschichteten Zellen, sodass die beschichteten Zellen dreidimensional geschichtet sind;
Bilden einer Grundschicht, die Typ-IV-Kollagen auf der Hautgewebeschicht durch Herstellen eines Kontakts zwischen Typ-IV-Kollagen und der Hautgewebeschicht beinhaltet; und
Bilden einer Epidermisschicht durch Anordnen von Epidermiszellen auf der Grundschicht,
wobei zumindest eine von der Hautgewebeschicht und der Epidermisschicht dendritische Zellen beinhaltet.

2. Verfahren nach Anspruch 1, wobei die Bildung der Hautgewebeschicht ein Kultivieren der beschichteten Zellen und der dendritischen Zellen umfasst, wobei eine Zelloberfläche mit einem Beschichtungsfilm beschichtet ist, der eine extrazelluläre Matrixkomponente enthält.

3. Verfahren nach einem der Ansprüche 1 oder 2, wobei die Bildung der Epidermisschicht das Anordnen von Epidermiszellen und dendritischen Zellen auf der Grundschicht umfasst.

4. Verfahren nach einem der Ansprüche 1 bis 3, wobei die Bildung der Grundschicht das Herstellen eines Kontakts abwechselnd zwischen Typ-IV-Kollagen und Laminin und der Hautgewebeschicht umfasst, sodass abwechselnd eine Typ-IV-Kollagen-Schicht und eine Lamininschicht auf der Hautgewebeschicht gebildet werden.

5. Verfahren nach einem der Ansprüche 1 bis 4, wobei die beschichteten Zellen Fibroblasten beinhalten, wobei die Zelloberfläche mit einem Beschichtungsfilm beschichtet ist, der eine extrazelluläre Matrixkomponente enthält.

6. Verfahren nach Anspruch 1, wobei die Bildung der Hautgewebeschicht ein Mischen von dendritischen Zellen und den beschichteten Zellen und ein anschließendes Kultivieren der gemischten dendritischen Zellen und beschichteten Zellen umfasst.

7. Verfahren nach Anspruch 6, wobei die Dichte der dendritischen Zellen 3 bis 200.000 Zellen/cm² beträgt.

8. Verfahren nach einem der Ansprüche 1 bis 7, wobei die Bildung der Hautgewebeschicht ein Mischen von Zellen zum Bilden des Gewebehilfsorgans mit den beschichteten Zellen oder ein Anordnen von Zellen zum Bilden des Gewebehilfsorgans zwischen den Zellschichten der beschichteten Zellen umfasst.

9. Verfahren nach Anspruch 8, wobei die Zellen zum Bilden des Gewebehilfsorgans eine Gefäßendothelzelle und/oder eine Lymphendothelzelle ist.

10. Verfahren nach einem der Ansprüche 1 bis 9, wobei die Dichte der beschichteten Zellen 1 × 10² Zellen/cm³ beträgt.

11. Künstliches Hautgewebe, hergestellt durch das Verfahren nach einem der Ansprüche 1 bis 10, wobei das künstliche Hautgewebe Folgendes umfasst:
eine Hautgewebeschicht, die eine extrazelluläre Matrixkomponente und geschichtete Zellen beinhaltet;
eine Grundschicht, die Typ-IV-Kollagen beinhaltet und auf der Hautgewebeschicht gebildet ist; und
eine Epidermisschicht, die auf der Grundschicht gebildet ist,
wobei die Hautgewebeschicht und gegebenenfalls die Epidermisschicht dendritische Zellen beinhalten,
wobei Zellen in der Hautgewebeschicht mittels der extrazellulären Matrixkomponente geschichtet sind, und
wobei die Hautgewebeschicht Lymphgefäße beinhaltet.

12. Künstliches Hautgewebe nach Anspruch 11, wobei in der Hautgewebeschicht das Verhältnis zwischen den dendritischen Zellen und anderen als den dendritischen Zellen zum Bilden der Hautgewebeschicht 1:99 bis 50:50 beträgt.

## Revendications

1. Procédé pour fabriquer un tissu de peau artificiel, comprenant :
la préparation de cellules revêtues dans lesquelles une surface de cellule est revêtue à l'aide d'un film de revêtement comprenant un composant de matrice extracellulaire,
la formation d'une couche de tissu dermique par la mise en culture des cellules revêtues, de sorte que les cellules revêtues soient disposées en couche tridimensionnellement ;
la formation d'une couche basale comportant du collagène de type IV sur la couche de tissu dermique en amenant en contact le collagène de type IV avec la couche de tissu dermique ; et
la formation d'une couche épidermique en agençant des cellules épidermiques sur la couche basale,
dans lequel au moins l'une de la couche de tissu dermique et de la couche épidermique comporte des cellules dendritiques.

2. Procédé selon la revendication 1, dans lequel la formation de la couche de tissu dermique comprend la mise en culture des cellules revêtues et des cellules dendritiques dans lesquelles une surface de cellule est revêtue à l'aide d'un film de revêtement contenant un composant de matrice extracellulaire.

3. Procédé selon la revendication 1 ou 2, dans lequel la formation de la couche épidermique comprend l'agencement de cellules épidermiques et de cellules dendritiques sur la couche basale.

4. Procédé selon l'une quelconque des revendications 1 à 3, dans lequel la formation de la couche basale comprend la mise en contact du collagène de type IV et de laminine avec la couche de tissu dermique en alternance de sorte qu'une couche de collagène de type IV et qu'une couche de laminine soient formées en alternance sur la couche de tissu dermique.

5. Procédé selon l'une quelconque des revendications 1 à 4, dans lequel les cellules revêtues comportent des fibroblastes dans lesquels la surface de cellule est revêtue à l'aide d'un film de revêtement contenant un composant de matrice extracellulaire.

6. Procédé selon la revendication 1, dans lequel la formation de la couche de tissu dermique comprend un mélange de cellules dendritiques et des cellules revêtues, puis la mise en culture des cellules dendritiques et des cellules revêtues mélangées.

7. Procédé selon la revendication 6, dans lequel la densité des cellules dendritiques est de 3 à 200 000 cellules/cm².

8. Procédé selon l'une quelconque des revendications 1 à 7, dans lequel la formation de la couche de tissu dermique comprend le mélange de cellules pour former l'organe auxiliaire de tissu avec les cellules revêtues, ou l'agencement de cellules pour former l'organe auxiliaire de tissu entre les couches de cellules des cellules revêtues.

9. Procédé selon la revendication 8, dans lequel la cellule pour former l'organe auxiliaire de tissu est une cellule endothéliale vasculaire et/ou une cellule endothéliale lymphatique.

10. Procédé selon l'une quelconque des revendications 1 à 9, dans lequel la densité des cellules revêtues est de 1 x 10² cellules/cm³.

11. Tissu de peau artificiel fabriqué par le procédé selon l'une quelconque des revendications 1 à 10, dans lequel ledit tissu de peau artificiel comprend :
une couche de tissu dermique qui comporte un composant de matrice extracellulaire et des cellules disposées en couche ;
une couche basale qui comporte du collagène de type IV et est formée sur la couche de tissu dermique ; et
une couche épidermique qui est formée sur la couche basale,
dans lequel la couche de tissu dermique et facultativement la couche épidermique comportent des cellules dendritiques,
dans lequel des cellules dans la couche de tissu dermique sont disposées en couche via le composant de matrice extracellulaire, et
dans lequel la couche de tissu dermique comporte des vaisseaux lymphatiques.

12. Tissu de peau artificiel selon la revendication 11, dans lequel le rapport entre les cellules dendritiques et les cellules autres que les cellules dendritiques pour former la couche de tissu dermique est de 1 : 99 à 50 : 50 dans la couche de tissu dermique.
